# EUROPÄISCHE PATENTANMELDUNG

(11) **EP 4 623 879 A1**
(43) Veröffentlichungstag der Anmeldung: **01.10.2025**
(21) Anmeldenummer: 25166302.7
(22) Anmeldetag: 26.03.2025
(51) Int. Cl.: A61F 13/471, A61F 5/453

(54) **HYGIENEARTIKEL FÜR MÄNNER**

(30) Priorität: 28.03.2024 DE 102024202995
(71) Anmelder: Kainz, Gerald, 74931 Lobbach (DE)
(72) Erfinder: Kainz, Gerald, 74931 Lobbach (DE)
(74) Vertreter: Ullrich & Naumann PartG mbB

(57) **Zusammenfassung**

Die Erfindung betrifft einen Hygieneartikel (1) für Männer zur Aufnahme von Urin beim Nachtröpfeln. Der Hygieneartikel (1) umfasst eine über den Penis (4) stülpbare Hülle (2) mit einem offenen Einsteckbereich (5) und einem endseitig geschlossenen Endbereich (3), wobei zumindest der Endbereich (3) flüssigkeitsabsorbierendes Material (9) umfasst oder daraus besteht.

## Beschreibung

Die Erfindung betrifft einen Hygieneartikel für Männer zur Aufnahme von Urin beim Nachtröpfeln.

Hygieneartikel der in Rede stehenden Art sind für den Anwendungsfall des Nachtröpfelns vorgesehen. Dabei handelt es sich um einen Vorgang, bei dem unmittelbar oder kurz nach dem Wasserlassen der in der Harn-Samen-Röhre verbleibende Urin, der sogenannte Post-Miktions-Tropfen, aus dem Penis heraustritt. Bei diesem Flüssigkeitsaustritt aus der Harn-Samen-Röhre handelt es sich nicht um einen Urinverlust bedingt durch eine Blasenschwäche, wie es bei der Inkontinez der Fall ist. Ursächlich für das Nachtrropfeln sind die anatomischen Gegebenheiten der Harn-Samen-Röhre, die eine S-förmige Krümmung aufweist, wodurch nach dem Wasserlassen eine geringe Menge an Urin in der Harn-Samen-Röhre verbleibt und erst verzögert aus dieser heraustritt. Darüber hinaus können pathogene Ursachen, beispielsweise Harnwegsinfektionen, eine Schwächung der Beckenbodenmuskulatur oder eine Verengung der Harn-Samen-Röhre das Nachtröpfeln verstärken.

Im Allgemeinen wird das Nachtröpfeln von der betroffenen Person als unangenehm oder peinlich empfunden, das Würdeempfinden ist herabgesetzt. Bei bestimmten Personenkreisen führt es darüber hinaus zu sozialen oder hygienischen Bedenken, wenn der ausgetretene Post-Miktions-Tropfen von außen wahrnehmbare Flecken hervorruft.

Hygieneartikel für Männer zur Aufnahme von Urin sind insbesondere als Hilfsmittel gegen Inkontinenz seit Jahren aus der Praxis bekannt. Derartige Hygieneartikel sind in unterschiedlicher Ausgestaltung auf dem Markt verfügbar, um austretenden Urin aus der Blase einer Person aufzunehmen. Zu nennen sind unter anderen Windeln, Einlagen und Urinalkondome. Sie alle haben gemeinsam, dass Sie für die Aufnahme eines größeren Flüssigkeitsvolumens ausgebildet sind. Die Harnblase einer erwachsenen männlichen Person besitzt ein Fassungsvermögen von ca. 350 bis 750 ml. Die Harn-Samen-Röhre hingegen hat mit einer durchschnittlichen Länge zwischen 20 und 25 cm und einem durchschnittlichen Durchmesser von 8 mm ein Volumen von ca. 10 ml. Entsprechend ist das durch den Hygieneartikel der in Rede stehenden Art aufzunehmende Volumen um ein Vielfaches kleiner.

Die aus der Praxis bekannten Hygieneartikel für Inkontinenz sind somit für die Aufnahme von Post-Miktions-Tropfen überdimensioniert und zumeist unhandlich. Darüber hinaus schränkt das Tragen einer Windel oder eines Urinalkondoms den Komfort der betroffenen Person im Alltag ein, nicht zuletzt wenn das Tragen eines solchen Hygieneartikels durch die Alltagsbekleidung von außen erkennbar ist.

Der vorliegenden Erfindung liegt daher die Aufgabe zugrunde, einen Hygieneartikel der eingangs genannten Art derart auszugestalten und weiterzubilden, dass eine einfache, bequeme und von außen nicht wahrnehmbare Vorrichtung zur Aufnahme von Urin beim Nachtröpfeln aus der Harn-Samen-Röhre geschaffen wird. Des Weiteren soll sich der Hygieneartikel von wettbewerblichen Produkten unterscheiden.

Erfindungsgemäß wird die voranstehende Aufgabe durch die Merkmale des Anspruches 1 gelöst. Danach umfasst der in Rede stehende Hygieneartikel für Männer zur Aufnahme von Urin beim Nachtröpfeln eine über den Penis stülpbare Hülle mit einem offenen Einsteckbereich und einem endseitig geschlossenen Endbereich. Zumindest der Endbereich umfasst flüssigkeitsabsorbierendes Material oder besteht daraus.

In erfindungsgemäßer Weise ist zunächst erkannt worden, dass durch geschickte Ausgestaltung des Hygieneartikesl die voranstehende Aufgabe auf überraschend einfache Weise gelöst wird.

Im Konkreten umfasst der Hygieneartikel eine Hülle, die am oberen Ende einen Einsteckbereich aufweist. Die Hülle wird im Einsteckbereich über den Penis gestülpt, sodass diese um den Penisschaft lösbar angeordnet ist. Somit kann der Hygieneartikel am Penis einer Person reversibel angebracht und wieder entfernt werden. Der beim Nachtröpfeln austretende Urin wird durch das flüssigkeitsabsorbierende Material aufgenommen, das zumindest im Endbereich der Hülle vorgesehen ist. Da beim Nachtröpfeln nur eine geringe Menge an Urin durch den Hygieneartikel aufgenommen werden muss, ist die Hülle dünnwandig und entspricht im Wesentlichen der Form des Penis bzw. ist diesem angepasst.

Folglich ist mit dem erfindungsgemäßen Hygieneartikel ein Hygieneartikel für Männer bereitgestellt, wonach eine einfache, bequeme und von außen nicht wahrnehmbare Vorrichtung zur Aufnahme von Urin beim Nachtröpfeln aus der Harn-Samen-Röhre geschaffen wird.

Der Begriff "Hülle" ist im weitesten Sinne zu verstehen und beschreibt eine künstlich geschaffene, physische Schicht, die insbesondere das distale Ende des Penis umgibt. Sie dient als Barriere, um das Austreten von Urin zu verhindern.

Die Hülle ist zumindest im Endbereich aus flüssigkeitsabsorbierendem Material ausgebildet. Darüber hinaus kann die Hülle mehrere Schichten umfassen, wobei eine Schicht aus einem flüssigkeitsundurchlässigem Material, insbesondere Latex, Vinyl, Silikon, oder hydrophilen Polymeren, gefertigt ist.

Der Begriff "flüssigkeitsabsorbierendes Material" ist im weitesten Sinne zu verstehen. Insbesondere beschreibt der Begriff poröse Feststoffe, die zur Aufnahme und zur Speicherung von Urin im freien Volumen des Materials geeignet sind, ohne dass diese ihre strukturelle Integrität verlieren.

An dieser Stelle sei angemerkt, dass flüssigkeitsabsorbierendes Material auch saugfähige Materialien umfasst. Diese Materialien ziehen Flüssigkeiten durch Kapillarkräfte, Porosität oder andere physikalische Mechanismen in ihre Struktur hinein. Saugfähige Materialien sind beispielsweise Baumwolle, Zellulose, Viskose oder Zellstoff aber auch Kunstfasern. Darüber hinaus sind Materialien mit einer schwammartigen oder porigen Innenstruktur oder durch ein textiles Flächengebilde, beispielsweise als Vliesstoff, Fasermatrix oder Gewebe, saugfähig.

Flüssigkeitsabsorbierende Materialien im Sinne der beanspruchten Lehre sind darüber hinaus Superabsorber. Das sind polymere Materialien, die eine außergewöhnlich hohe Menge an Flüssigkeit im Verhältnis zu ihrem eigenen Gewicht absorbieren und zurückhalten können. Diese Polymere quellen auf, wenn sie mit Flüssigkeit in Kontakt kommen, und bilden ein stabiles Hydrogel. Superabsorber sind beispielsweise Polyacrylate, insbesondere Natriumpolyacrylat, Polyvinylalkohol, Alginate oder stärke- bzw. cellulosebasierte Superabsorber.

Flüssigkeitsabsorbierende Materialien können aus einer Kombination mehrerer Stoffe in Form eines Verbundmaterials ausgeführt sein. Beispielsweise kann das Verbundmaterial eine flüssigkeitsdurchlässige und/oder -verteilende Oberflächenschicht, eine Absorptionsschicht und eine flüssigkeitsundurchlässige Stützschicht aufweisen. Die Absorptionsschicht kann beispielsweise als eine Fasermatrix oder als Vliesstoff ausgebildet sein, in der bzw. in dem ein Superabsorber, vorzugsweise als Granulat oder pulverförmig, eingebettet ist.

Auch ist es denkbar, die Hülle aus einem Vlies oder aus weichem Stoff (z.B. Textil) zu fertigen, wobei die Innenseite saugfähig sein sollte. Zudem kann die Hülle (zumindest außen) undurchlässig für Feuchtigkeit sein.

Es sei angemerkt, dass im Sinne der beanspruchten Lehre der Begriff "Urin" sämtliche auf natürliche Weise gebildete Ausscheidungsprodukte aus dem Penis, insbesondere die in der Harnflüssigkeit enthaltenen gelösten Stoffe, aber auch Samenflüssigkeit und krankheitsbedingte Ausscheidungsprodukte aus dem Harnsystem umfasst.

In vorteilhafter Weise umfasst das flüssigkeitsabsorbierende Material Superabsorber. Vorzugsweise ist der Superabsorber als Granulat in einer Matrix aus textilem Flächengebilde, beispielsweise als Vliesstoff, Fasermatrix oder Gewebe, eingebettet. Der Superabsorber erhöht aufgrund seiner saugfähigen Eigenschaften die Aufnahmekapazität des flüssigkeitsabsorbierenden Materials. Somit lässt sich die Schichtdicke der Hülle bei Verwendung von Superabsorbern verringern, was die äußere Wahrnehmbarkeit des Hygieneartikels reduziert.

In einer vorteilhaften Ausführungsform ist der Hygieneartikel biologisch abbaubar.

Dazu umfasst der Hygieneartikel ausschließlich natürliche Materialien, vorzugsweise Zellulose, Zellstoff oder Baumwolle, und/oder synthetische Biopolymere.

Der Ausdruck "biologisch abbaubar" ist im weitesten Sinne zu verstehen und beschreibt die Eigenschaft eines Materials, durch natürliche Prozesse, typischerweise durch die Aktion von Mikroorganismen wie Bakterien, Pilzen und Algen, in seine grundlegenden Bestandteile zerlegt und somit aus der Umwelt entfernt zu werden, ohne schädliche Rückstände zu hinterlassen. Biologisch abbaubare Hygieneartikel verringern somit die Abfallbelastung.

In einer Weiterbildung der Erfindung weist die Hülle an ihrer Innenseite zumindest teilweise eine adhäsive Oberfläche auf, die einen temporären Halt ermöglicht. Die adhäsive Oberfläche bewirkt einen sicheren Sitz des Hygieneartikels um den Penisschaft.

Eine adhäsive Oberfläche besitzt die Eigenschaft, an anderen Materialien oder Oberflächen durch verschiedene physikalische und chemische Wechselwirkungen zu haften. Im Gegensatz zu mechanischem Haften erfolgt die Bindung bei adhäsiven Oberflächen auf molekularer Ebene. Die adhäsive Wirkung wird durch Klebstoffe, Harze, Haftgel, Gummi oder silikonbasierte Adhäsive ermöglicht.

Vorzugsweise ist die adhäsive Oberfläche eine Klebefläche, insbesondere Klebestreifen. Die Klebefläche ermöglicht eine sichere, reversible Haftung des Hygieneartikels am Penis einer Person.

Alternativ oder in Kombination dazu weist die Hülle an ihrer Innenseite zumindest teilweise eine rutschhemmende Oberfläche auf, die einen temporären Halt ermöglicht. Eine rutschhemmende Oberfläche erhöht den Widerstand der Hülle gegen das Verrutschen durch eine erhöhte Reibkraft, die die Hülle auf den Penisschaft einer Person ausübt.

Vorzugsweise ist die rutschhemmende Oberfläche eine profilierte Fläche, insbesondere ein geriffeltes Muster, Noppen oder Rillen. Durch die Querschnittsverengung liegt die Hülle reibschlüssig um Penisschaft einer Person an, ohne diesen einzuschnüren. Dadurch ist ein Verrutschen oder ein ungewolltes Herabgleiten des Hygieneartikels vom Penis nicht möglich.

In einer Ausgestaltung des erfindungsgemäßen Hygieneartikels weist die Hülle zumindest im Endbereich eine flüssigkeitsundurchlässige Schicht umfasst. Somit wird verhindert, dass Flüssigkeit, die nicht durch das flüssigkeitsabsorbierende Material aufgenommen wird nicht aus der Hülle austritt und folglich zu sichtbaren Flecken auf der Hose führt.

Des Weiteren ist es von Vorteil, wenn die Hülle atmungsaktives Material umfasst. Atmungsaktive Materialien ermöglichen einen Luftaustausch durch die Hülle, während es den Urin in der Hülle zurückhält. Dadurch wird der Komfort beim Tragen des Hygieneartikels erhöht. Atmungsaktive Materialien sind vorzugsweise Vliesstoffe aus Polyester oder Polypropylen, Silikonfolien oder Polyurethanfilme.

In vorteilhafter Weise umfasst die Hülle geruchsneutralisierendes Material. Dadurch kann Diskretion und Komfort für den Nutzer gewährleistet werden. Geruchsneutralisierende Materialien sind beispielsweise Aktivkohle, vorzugsweise als Pulver, metallische Zusätze, insbesondere Silberionen, Zinkoxid und Cyclodextrine.

In einer Weiterbildung der Erfindung weist die Hülle hautfreundliches, hypoallergenes oder desinfizierendes Material auf. Dadurch können bei regelmäßiger oder längerer Benutzung des Hygieneartikels Hautirritationen oder Infektionen am Penis im Bereich unter der Hülle vermieden und der Tragekomfort erhöht werden.

In einer vorteilhaften Ausführungsform ist die Hülle dehnbar. Die Dehnbarkeit ermöglicht eine bessere Anpassung an die Form und Größe des Penis und ermöglicht einen gesteigerten Komfort beim Tragen.

In einer Ausgestaltung des erfindungsgemäßen Hygieneartikels kann dieser, vorzugsweise im Einsteckbereich eine Klammer aufweisen, die um den Penisschaft gelegt wird und einen sanften Druck auf den Penis auslöst, so dass ein temporär sicherer Sitz des Hygieneartikels gewährleistet ist.

In einer Weiterbildung der Erfindung weist der Hygieneartikel im Einsteckbereich ein umlaufendes Halteband aus. Dieses Halteband ist insbesondere ein Gummiband, dass sich reibschlüssig um den Penisschaft legt. Alternativ ist das Halteband ein Faden. Durch das Halteband wird ein sicherer, rutschfester Sitz des Hygieneartikels ermöglicht.

In einer Ausgestaltung des erfindungsgemäßen Hygieneartikels ist die Hülle im Endbereich endseitig, vorzugsweise durch eine geriffelte Falz, verpresst. Bei Produktion des Hygieneartikels, beispielsweise als Wegwerfprodukt aus Zellstoff, kann durch diese Ausführungsform Material und Zeit eingespart werden, wodurch eine kostengünstige Herstellung des Hygieneartikels ermöglicht wird.

Alternativ oder in Kombination dazu ist der erfindungsgemäße Hygieneartikel längsseitig, vorzugsweise durch eine geriffelte Falz, verpresst.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: erfindungsgemäßen Hygieneartikel für Männer in Querschnitt;
- Fig. 2: erfindungsgemäßen Hygieneartikel mit profiliertem Wandungsabschnitt im Einsteckbereich;
- Fig. 3: Hygieneartikel mit umlaufenden Gummiband im Einsteckbereich; und
- Fig. 4: Hygieneartikel mit verpresstem Endstück.

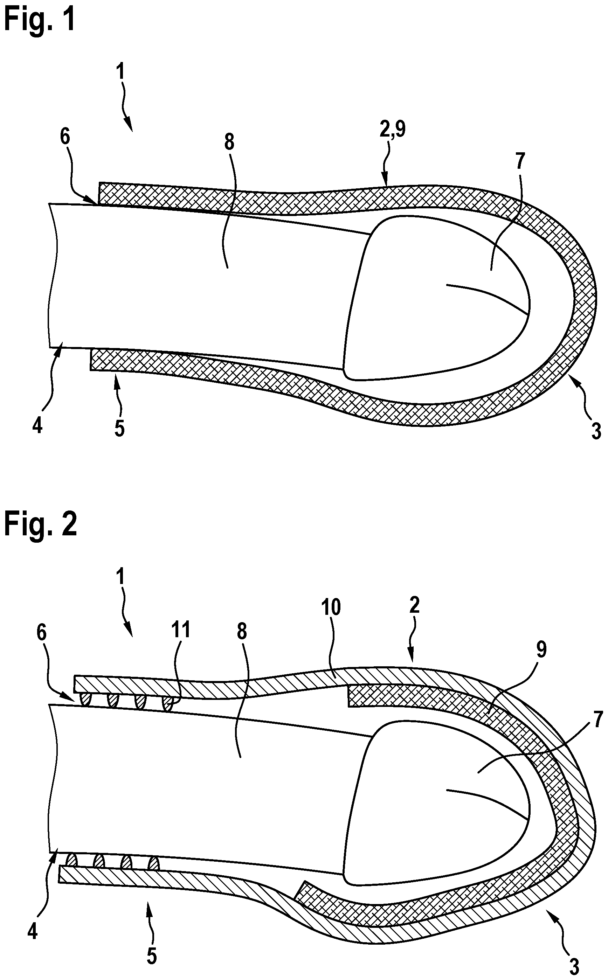

Fig. 1 zeigt in einer schematischen Querschnittsdarstellung den erfindungsgemäßen Hygieneartikel 1. Der Hygieneartikel 1 weist eine Hülle 2 auf, die im unteren Endbereich 3 den Penis 4 am distalen Ende vollständig umschließt. Im oberen Einsteckbereich 5 ist eine Öffnung 6 vorgesehen, durch die der Penis 4 in die Hülle 2 eingreift, sodass die Hülle 2 zumindest über den Peniskopf 7 gestülpt ist. Im Einsteckbereich 5 liegt die Hülle 2 um den Penisschaft 8 reibschlüssig an, sodass der Hygieneartikel 1 einen sicheren Sitz hat. Der Hygieneartikel 1 ist zerstörungsfrei vom Penis 4 lösbar.

Die Hülle 2 ist aus einem flüssigkeitsabsorbierenden Material 9, vorzugsweise Zellstoff oder Vliesstoff, ausgeführt um austretenden Urin aufzunehmen. Des Weiteren kann die Hülle 2 atmungsaktiv, geruchsneutralisierend, hautfreundliches, hypoallergen und/oder desinfizierend sein. Darüber hinaus ist die Hülle 2 zumindest in dem Maße dehnbar, dass die Hülle 2 über den Peniskopf 7 gestülpbar ist und reibschlüssig um den Penisschaft 8 anliegt.

Alternativ oder in Kombination dazu weisen der Einsteckbereich 5 und der Endbereich 3 unterschiedliche Durchmesser auf.

Alternativ oder in Kombination dazu weist die Hülle 2 eine wellenförmige, sternartige oder zickzackförmige Faltung auf, so dass der der Querschnitt flexibel an den Umfang des Penisschaft anpasst.

Fig. 2 zeigt in einer schematischen Querschnittsdarstellung eine weitere Ausführungsform des erfindungsgemäßen Hygieneartikels 1. Die Hülle 2 umfasst mehrere Schichten. Die äußere Schicht ist aus einem flüssigkeitsundurchlässigem Material 10 ausgeführt. Darüber hinaus kann die Schicht atmungsaktiv sein. Im Endbereich 3 der Hülle 2 ist auf der Innenseite eine innenliegende Schicht angeordnet, die aus einem flüssigkeitsabsorbierenden Material 9 besteht.

Des Weiteren kann die Hülle 2 geruchsneutralisierend, hautfreundliches, hypoallergen und/oder desinfizierend sein.

Auf der Innenseite des Einsteckbereichs 5 ist eine Profilierung 11 als rutschhemmende Oberfläche vorgesehen. Diese verengt punktuell den inneren Querschnitt der Hülle 2 um den Penisschaft 8, so dass der Widerstand gegen das Verrutschen des Hygieneartikels erhöht ist.

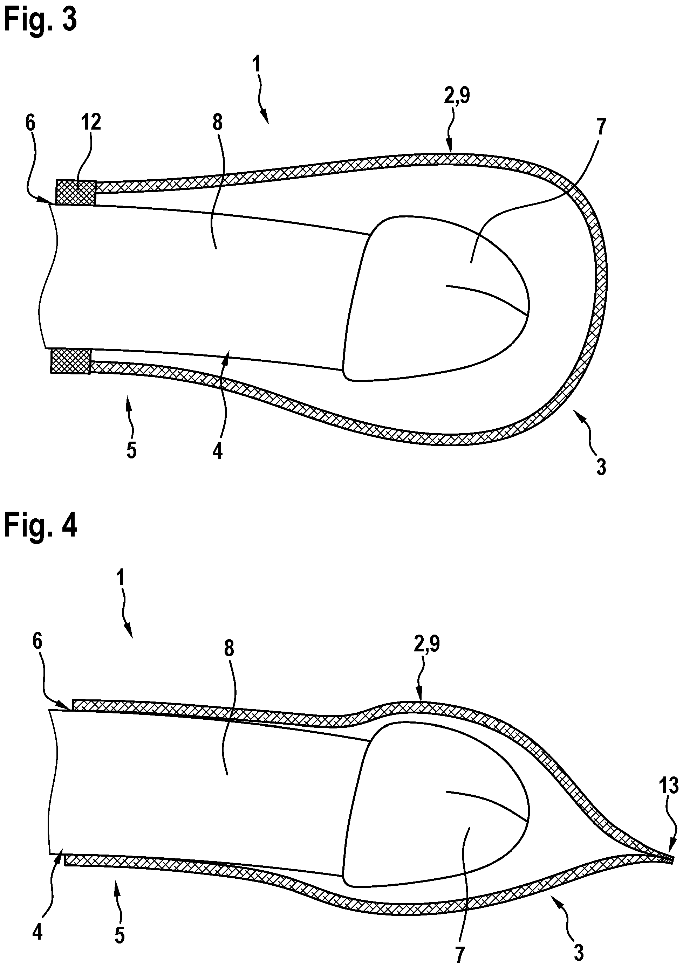

Fig. 3 zeigt eine dritte Ausführungsform des erfindungsgemäßen Hygieneartikels 1. Die Hülle weist im Einsteckbereich 5 ein umlaufendes Gummiband 12 auf, dass reibschlüssig um den Penisschaft 8 anliegt. Dadurch ist ein sicherer Sitz des Hygieneartikels 1 am Penis 4 möglich. Die Hülle 2 hat somit auch einen sicheren Sitz am Penis 4, ohne dass dieser über einen größeren Abschnitt am Penisschaft 8 anliegen muss. Somit kann allgemein ein größerer Durchmesser der Hülle 2 gewählt werden, sodass der Tragekomfort des Hygieneartikels 1 gesteigert ist.

Fig. 4 zeigt eine Ausführungsform des erfindungsgemäßen Hygieneartikels 4, bei dem die Hülle 2 im Endbereich 3 mit einer geriffelten Falz 13 endseitig verpresst ist. Alternativ oder darüber hinaus ist die Hülle in Längsrichtung mit einer geriffelten Falz 13 verpresst.

Hinsichtlich weiterer vorteilhafter Ausgestaltungen des erfindungsgemäßen Hygieneartikels wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele des erfindungsgemäßen Hygieneartikels lediglich zur Er-örterung der beanspruchten Lehre dienen, diese jedoch nicht auf die Ausführungsbeispiele einschränken.

### Bezugszeichenliste

- 1: Hygieneartikel
- 2: Hülle
- 3: Endbereich
- 4: Penis
- 5: Einsteckbereich
- 6: Öffnung
- 7: Peniskopf
- 8: Penisschaft
- 9: Flüssigkeitsabsorbierendes Material
- 10: Flüssigkeitsundurchlässiges Material
- 11: Profilierung
- 12: Gummiband
- 13: Falz

## Patentansprüche

1. Hygieneartikel (1) für Männer zur Aufnahme von Urin beim Nachtröpfeln, umfassend eine über den Penis (4) stülpbare Hülle (2) mit einem offenen Einsteckbereich (5) und einem endseitig geschlossenen Endbereich (3), wobei zumindest der Endbereich (3) flüssigkeitsabsorbierendes Material (9) umfasst oder daraus besteht.

2. Hygieneartikel (1) nach Anspruch 1 **dadurch gekennzeichnet, dass** das feuchtigkeitsabsorbierende Material (9) einen Superabsorber, insbesondere ein Polyacrylat, umfasst.

3. Hygieneartikel (1) nach Anspruch 1 oder 2 **dadurch gekennzeichnet, dass** der Hygieneartikel (1) biologisch abbaubar ist.

4. Hygieneartikel (1) nach einem der Ansprüche 1 bis 3 **dadurch gekennzeichnet, dass** die Innenseite der Hülle (2) zumindest teilweise eine temporär adhäsive und/oder rutschhemmende Oberfläche aufweist.

5. Hygieneartikel (1) nach Anspruch 4 **dadurch gekennzeichnet, dass** die adhäsive Oberfläche eine Klebefläche, insbesondere Klebestreifen, ist.

6. Hygieneartikel (1) nach Anspruch 4 **dadurch gekennzeichnet, dass** die rutschhemmende Oberfläche eine profilierte Fläche ist.

7. Hygieneartikel (1) nach einem der Ansprüche 1 bis 6 **dadurch gekennzeichnet, dass** die Hülle (2) zumindest im Endbereich eine flüssigkeitsundurchlässige Schicht (10) umfasst.

8. Hygieneartikel (1) nach einem der Ansprüche 1 bis 7 **dadurch gekennzeichnet, dass** die Hülle (2) atmungsaktives, geruchsneutralisierendes, hautfreundliches, hypoallergenes und/oder desinfizierenden Material umfasst.

9. Hygieneartikel (1) nach einem der Ansprüche 1 bis 8 **dadurch gekennzeichnet, dass** die Hülle (2) dehnbar ist.

10. Hygieneartikel (1) nach einem der Ansprüche 1 bis 9 **dadurch gekennzeichnet, dass** der Hygieneartikel (1) eine Klammer umfasst.

11. Hygieneartikel (1) nach einem der Ansprüche 1 bis 10 **dadurch gekennzeichnet, dass** der Einsteckbereich (5) ein umlaufendes Halteband, insbesondere ein Gummiband (12), aufweist.

12. Hygieneartikel (1) nach einem der Ansprüche 1 bis 11 **dadurch gekennzeichnet, dass** die Hülle (2) im Endbereich endseitig verpresst ist.
